(19) Europäisches Patentamt
European Patent Office
Office européen des brevets

(11) EP 1 559 399 A1

(12) **DEMANDE DE BREVET EUROPEEN**

(43) Date de publication:
**03.08.2005 Bulletin 2005/31**

(51) Int Cl.[7]: **A61K 7/06**

(21) Numéro de dépôt: **05300070.9**

(22) Date de dépôt: **28.01.2005**

(84) Etats contractants désignés:
**AT BE BG CH CY CZ DE DK EE ES FI FR GB GR HU IE IS IT LI LT LU MC NL PL PT RO SE SI SK TR**
Etats d'extension désignés:
**AL BA HR LV MK YU**

(30) Priorité: **29.01.2004 FR 0450164**

(71) Demandeur: **L'OREAL**
**75008 Paris (FR)**

(72) Inventeurs:
- **Barbarat, Philippe**
**92270, BOIS-COLOMBES (FR)**
- **Andrean, Hervé**
**75014, PARIS (FR)**
- **Rayee, Chrystelle**
**93600, AULNAY-SOUS-BOIS (FR)**

(74) Mandataire: **Catherine, Alain et al**
**Cabinet Harlé & Phélip**
**7, rue de Madrid**
**75008 Paris (FR)**

(54) **Utilisation de dérivés du 2-iminothiolane dans les produits de soins capillaires**

(57) Procédé pour renforcer les cheveux, et procédé pour améliorer la compatibilité d'un traitement de mise en forme des cheveux avec un traitement cosmétique ultérieur différent, lesdits procédés comprenant une étape de traitement des cheveux pour une composition de pH neutre comprenant, dans un milieu cosmétiquement acceptable, au moins un dérivé du 2-iminothiolane de formule (1) :

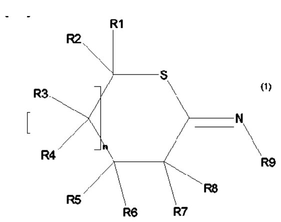

(1)

où
R1 à R8 représentent indépendamment soit un atome d'hydrogène, soit une chaîne hydrocarbonée linéaire ou ramifiée en $C_1$-$C_4$, présentant éventuellement une ou plusieurs insaturations, éventuellement substituée par un ou plusieurs groupes choisis parmi les groupes thiol, thioalkyle en $C_1$-$C_4$, alkylthiol en $C_1$-$C_4$, N,N-dialkylamine en $C_2$-$C_8$, alkylcarbonylthiol en $C_2$-$C_4$, ou N-alkylcarbonylamine en $C_2$-$C_4$, soit un groupe aryle en $C_6$ éventuellement substitué par un ou plusieurs groupes choisis parmi les groupes halogéno, thiol oualkyle linéaire ou ramifié en $C_1$-$C_4$,
R9 désigne un atome d'hydrogène ou une chaîne hydrocarbonée linéaire ou ramifiée en $C_1$-$C_4$,
n est égal à 0 ou 1 ;
et/ou au moins un sel hydrosoluble desdits dérivés de formule (1).

EP 1 559 399 A1

## Description

L'invention se rapporte d'une manière générale à des procédés de traitement des cheveux, utilisant à pH neutre des dérivés du 2-iminothiolane (2-IT).

En particulier, l'invention se rapporte à un procédé pour renforcer les cheveux, qui également améliore la compatibilité d'un traitement de mise en forme des cheveux avec un traitement cosmétique ultérieur différent, ce procédé comprenant le traitement des cheveux avec une composition de pH neutre contenant au moins un dérivé du 2-iminothiolane.

Les dérivés du 2-iminothiolane sont connus dans l'art antérieur notamment comme agents de couplage. Ainsi, par exemple, le brevet US 5,395,946 décrit l'utilisation des dérivés du 2-iminothiolane comme agent de couplage dans la réaction entre une protéine et un radionucléide.

Dans la publication Textile Res. J.72,285-289 (2002), il est montré qu'un traitement de la laine au 2-iminothiolane à pH neutre favorise la disparition de plis indésirables.

Mais aucun document ne décrit l'utilisation des dérivés du 2-iminothiolane pour renforcer les cheveux ou pour améliorer la compatibilité d'un traitement de mise en forme avec un traitement cosmétique ultérieur différent.

Par ailleurs, concernant la mise en forme des cheveux, et en particulier la déformation permanente, la technique généralement utilisée consiste, dans un premier temps, à réaliser l'ouverture des liaisons disulfures -S-S- de la kératine (cystine) en appliquant sur les cheveux préalablement mis sous tension (bigoudis et autres) une composition réductrice (étape de réduction) puis, de préférence après avoir rincé la chevelure ainsi traitée, à reconstituer dans un second temps lesdites liaisons disulfures en appliquant sur les cheveux toujours sous tension une composition oxydante (étape d'oxydation, dite aussi de fixation) de façon à donner finalement aux cheveux la forme recherchée.

Les compositions réductrices utilisables pour la mise en oeuvre de la première étape d'un tel procédé classique de déformation permanente des cheveux (procédé en deux temps) contiennent généralement des composés thiolés, tels que l'acide thioglycolique ou la cystéine, ou des composés générant des ions hydroxydes, tels que la soude ou l'hydroxyde de guanidine (généré in situ ou non) par exemple.

Cependant, une telle technique ne donne pas entièrement satisfaction.

Ainsi, par exemple, si une coloration est appliquée sur des cheveux permanentés selon la technique décrite précédemment, la couleur obtenue est différente de la couleur normalement obtenue sur des cheveux naturels non permanentés.

Il a été proposé de remplacer l'acide thioglycolique par le 2-iminothiolane pour limiter la dégradation chimique des cheveux.

Ainsi, le brevet US 4,263,277 décrit l'utilisation d'une composition de pH compris entre 9,2 et 9,5 comprenant du 2-iminothiolane, pour la déformation permanente des cheveux.

D'autre part, dans la publication J. Appl. Polym. Science 90, 3646-3651 (2003), il est montré qu'un traitement du cheveu au 2-iminothiolane à pH égal à 8 ou 9 conduit à une mise en forme durable de la fibre.

Mais rien n'est dit sur l'utilisation des dérivés du 2-iminothiolane à pH neutre pour améliorer la compatibilité d'un traitement de mise en forme des cheveux avec un traitement cosmétique ultérieur différent, par exemple un traitement de coloration des cheveux.

L'invention a donc pour but de fournir d'une part un procédé pour renforcer les cheveux, et d'autre part un procédé pour améliorer la compatibilité d'un traitement de mise en forme des cheveux avec un traitement cosmétique ultérieur différent.

Par traitement cosmétique ultérieur différent, on entend tout traitement autre qu'un traitement de mise en forme des cheveux, notamment un traitement de coloration des cheveux.

Ainsi, l'invention a donc d'abord pour objet un procédé pour renforcer les cheveux, comprenant une étape de traitement des cheveux par une composition de pH neutre comprenant, dans un milieu cosmétiquement acceptable, au moins un dérivé du 2-iminothiolane de formule (1) :

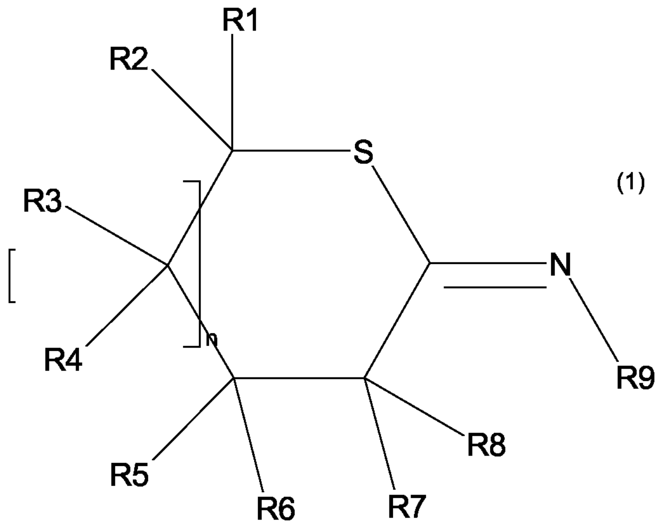

(1)

où

R1 à R8 représentent indépendamment soit un atome d'hydrogène, soit une chaîne hydrocarbonée, linéaire ou ramifiée en $C_1$-$C_4$, présentant éventuellement une ou plusieurs insaturations, éventuellement substituée par un ou plusieurs groupes choisis parmi les groupes thiol, thioalkyle en $C_1$-$C_4$, alkylthiol en $C_1$-$C_4$, N,N-dialkylamine en $C_2$-$C_8$, alkylcarbonylthiol en $C_2$-$C_4$, ou N-alkylcarbonylamine en $C_2$-$C_4$, soit un groupe aryle en $C_6$ éventuellement substitué par un ou plusieurs groupes choisis parmi les groupes halogéno, thiol ou alkyle linéaire ou ramifié en $C_1$-$C_4$,

R9 désigne un atome d'hydrogène ou une chaîne hydrocarbonée linéaire ou ramifiée en $C_1$-$C_4$,

n est égal à 0 ou 1 ;

et/ou au moins un sel hydrosoluble desdits dérivés de formule (1).

L'utilisation des dérivés de formule (1) et de leurs sels hydrosolubles, en augmentant la rigidité des cheveux, conduit à un renforcement des cheveux et permet d'accroître le volume de la chevelure.

Le procédé selon l'invention peut être mis en oeuvre indépendamment de tout autre traitement capillaire.

Mais il peut également avantageusement être mis en oeuvre en association avec un autre traitement capillaire, c'est-à-dire avant, pendant ou après ledit traitement.

Cet autre traitement capillaire peut être tout traitement capillaire classique, par exemple un shampooing, un après-shampooing, une mise en forme permanente ou semi-permanente, un défrisage ou un coiffage.

L'invention a encore pour objet un procédé pour améliorer la compatibilité d'un traitement de mise en forme des cheveux avec un traitement cosmétique ultérieur, autre qu'un traitement de mise en forme des cheveux, comprenant une étape initiale de réduction et une étape ultérieure de fixation, ledit procédé consistant à remplacer l'étape initiale de réduction par une étape de traitement avec une composition de pH neutre comprenant, dans un milieu cosmétiquement acceptable, au moins un dérivé du 2-iminothiolane de formule (1) :

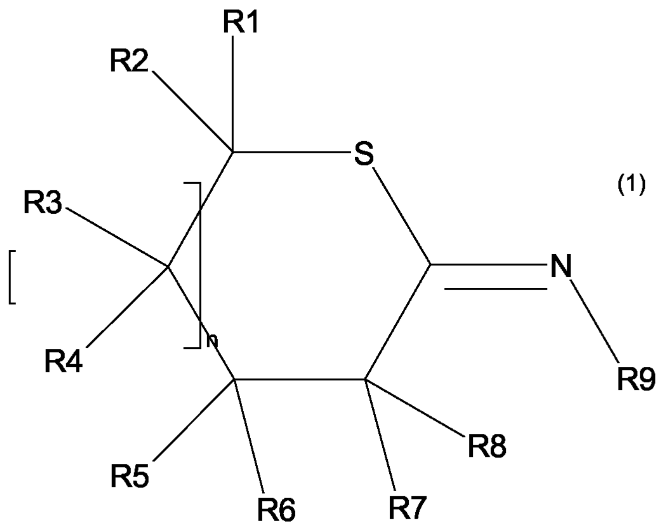

(1)

où

R1 à R8 représentent indépendamment soit un atome d'hydrogène, soit une chaîne hydrocarbonée, linéaire ou ramifiée en $C_1$-$C_4$, présentant éventuellement une ou plusieurs insaturations, éventuellement substituée par un ou plusieurs groupes choisis parmi les groupes thiol, thioalkyle en $C_1$-$C_4$, alkylthiol en $C_1$-$C_4$, N,N-dialkylamine en $C_2$-$C_8$, alkylcarbonylthiol en $C_2$-$C_4$, ou N-alkylcarbonylamine en $C_2$-$C_4$, soit un groupe aryle en $C_6$ éventuellement substitué par un ou plusieurs groupes choisis parmi les groupes halogéno, thiol ou alkyle linéaire ou ramifié en $C_1$-$C_4$,

R9 désigne un atome d'hydrogène ou une chaîne hydrocarbonée linéaire ou ramifiée en $C_1$-$C_4$,

n est égal à 0 ou 1 ;

et/ou au moins un sel hydrosoluble desdits dérivés de formule (1).

Comme expliqué précédemment, le procédé selon l'invention présente une étape ultérieure de fixation.

Cette étape de fixation peut se faire soit par mise en contact des cheveux avec l'oxygène de l'air, soit par application d'une composition oxydante comprenant au moins un agent oxydant.

Généralement, l'agent oxydant est choisi parmi l'eau oxygénée et les bromates alcalins, de préférence l'eau oxygénée.

L'étape de traitement et l'étape de fixation sont généralement séparées par un temps de pose : on laisse poser la composition comprenant le ou les dérivés de formule (1) et/ou leurs sels hydrosolubles pendant une durée généralement comprise entre 10 minutes et 3 heures, à une température de préférence comprise entre 20°C et 70°C.

Ainsi, l'étape de traitement par un ou plusieurs dérivés de formule (1) et/ou leurs sels hydrosolubles, et l'étape de fixation, conduisent à une mise en forme permanente des cheveux.

Comme indiqué précédemment, ce procédé selon l'invention permet d'améliorer la compatibilité du traitement de mise en forme des cheveux obtenue avec un traitement cosmétique ultérieur, autre qu'un traitement de mise en forme des cheveux.

En particulier, le procédé selon l'invention est particulièrement avantageux lorsque le traitement cosmétique ultérieur est une coloration d'oxydation.

En effet, les traitements classiques de mise en forme permanente des cheveux, en particulier ceux utilisant l'acide thioglycolique et l'eau oxygénée à pH alcalin, sont connus pour pouvoir engendrer une dégradation de la fibre capillaire. Une conséquence de cette dégradation est, par exemple, l'amplification de la montée de la couleur lors de l'application d'une coloration d'oxydation sur cheveux permanentés : la couleur obtenue est beaucoup plus intense sur les cheveux permanentés que sur les cheveux naturels. La différence de montée de la couleur sur cheveux naturels et sur cheveux permanentés produit alors sur tête un effet non esthétique.

Ainsi, lorsque l'on met en forme les cheveux en utilisant le procédé selon l'invention, c'est-à-dire en effectuant une étape de traitement des cheveux par une composition de pH neutre comprenant au moins un dérivé de formule (1) et/ou un de leurs sels hydrosolubles, et en effectuant une étape de fixation, on limite la montée de la couleur lors d'une coloration d'oxydation ultérieure et on réduit ainsi l'écart entre la montée en couleur sur cheveux permanentés et la montée en couleur sur cheveux naturels.

La coloration d'oxydation est généralement effectuée à l'aide d'un ou plusieurs colorants d'oxydation.

Les colorants d'oxydation sont des composés ou mélanges de composés qui, en présence d'un oxydant, par exemple l'oxygène de l'air ou l'eau oxygénée, s'oxydent pour donner un composé ou un mélange de composés colorés.

Les colorants d'oxydation sont généralement choisis parmi les bases d'oxydation, les orthodiphénols, les coupleurs, et leurs mélanges.

Les bases d'oxydation, de type para ou ortho, sont des composés qui ne sont pas des colorants en eux-mêmes, mais forment un colorant par un processus de condensation oxydative, soit sur eux-mêmes, soit en présence d'un coupleur ou modificateur. Ils comportent des groupements fonctionnels, soit deux groupements amino, soit un groupement amino et un groupement hydroxy en position para ou ortho l'un par rapport à l'autre.

La nature de ces bases d'oxydation n'est pas critique. Elles peuvent notamment être choisies parmi les ortho- et para-phénylènediamines, les bases doubles, les ortho- et para-aminophénols, les bases hétérocycliques ainsi que les sels d'addition de tous ces composés avec un acide.

Les orthodiphénols sont des composés comportant au moins un cycle aromatique dont au moins deux carbones consécutifs portent un groupe hydroxyle. De préférence, le cycle aromatique est un cycle benzénique ou un cycle aromatique condensé.

Le cycle aromatique peut être un cycle aromatique condensé contenant éventuellement un ou plusieurs hétéroatomes, tel que le naphtalène, le tétrahydronaphtalène, l'indane, l'indène, l'anthracène, le phénanthrène, l'indole, l'isoindole, l'indoline, l'isoindoline, le benzofuranne, le dihydrobenzofuranne, le chromane, l'isochromane, le chromène, l'isochromène, la quinoléine, la tétrahydroquinoléine et l'isoquinoléine.

Une autre classe de colorants d'oxydation est constituée par les coupleurs, comme expliqué précédemment. Les coupleurs peuvent être choisis parmi ceux classiquement utilisés en teinture d'oxydation et notamment parmi les méta-aminophénols, les méta-phénylènediamines, les métadiphénols, les naphtols et les coupleurs hétérocycliques tels que par exemple les dérivés indoliques, les dérivés indoliniques, le sésamol et ses dérivés, les dérivés pyridiniques, les dérivés pyrazolotriazoles, les pyrazolones, les indazoles, les benzimidazoles, les benzothiazoles, les benzoxazoles, les 1,3-benzodioxoles, les quinolines et leurs sels d'addition avec un acide, ces composés étant différents des composés orthodihydroxylés de l'invention.

Ces coupleurs sont plus particulièrement choisis parmi le 2,4-diamino 1-(β-hydroxyéthyloxy) benzène, le 2-méthyl 5-amino phénol, le 5-N-(β-hydroxyéthyl)amino 2-méthyl phénol, le 3-amino phénol, le 1,3-dihydroxy benzène, le 1,3-dihydroxy 2-méthyl benzène, le 4-chloro 1,3-dihydroxy benzène, le 2-amino 4-(β-hydroxyéthylamino) 1-méthoxy benzène, le 1,3-diamino benzène, le 1,3-bis-(2,4-diaminophénoxy) propane, le sésamol, le 1-amino 2-méthoxy 4,5-méthylènedioxy benzène, l'α-naphtol, le 6-hydroxy indole, le 4-hydroxy indole, le 4-hydroxy N-méthyl indole, la 6-hydroxy indoline, la 2,6-dihydroxy 4-méthyl pyridine, le 1-H 3-méthyl pyrazole 5-one, le 1-phényl 3-méthyl pyrazole 5-one, la 2-amino 3-hydroxypyridine, le 3,6-diméthyl-pyrazolo-[3,2-c]-1,2,4-triazole, le 2,6-diméthyl-pyrazolo-[1,5-b]-1 ,2,4-triazole et leurs sels d'addition avec un acide.

La quantité de colorants d'oxydation dans la composition de coloration doit être suffisante pour obtenir une coloration visible. Cette quantité peut varier dans de larges mesures en fonction de la nature du colorant d'oxydation et de l'intensité voulue pour la coloration.

En général, on obtiendra une coloration convenable lorsque la quantité de colorant est telle que la teneur en colorant dans la composition de coloration finale est d'au moins 0,1 micromole, de préférence d'au moins 1 micromole par millilitre de composition finale.

Typiquement, la quantité de colorant d'oxydation dans la composition de coloration varie de 1 mM à 10 mM par litre et est généralement de l'ordre de 5 mM par litre.

En faisant varier la nature des différents colorants d'oxydation et leurs proportions dans la composition, on peut faire varier la couleur de la composition de coloration finale. On obtient ainsi une palette de couleurs.

Les compositions de coloration capillaire peuvent, outre les colorants d'oxydation, renfermer un ou plusieurs colorants directs, notamment pour modifier les nuances en les enrichissant de reflets. Ces colorants directs peuvent notamment alors être choisis parmi les colorants nitrés, azoïques ou anthraquinoniques, neutres, cationiques ou anioniques dans la proportion pondérale d'environ 0,001 à 20% et de préférence de 0,01 à 10% du poids total de la composition.

Les caractéristiques décrites dans la description qui suit s'appliquent aux deux procédés objet de la présente invention, c'est-à-dire au procédé pour augmenter la rigidité des cheveux et au procédé pour améliorer la compatibilité d'un traitement de mise en forme des cheveux avec un traitement cosmétique ultérieur différent.

Comme décrit précédemment, les procédés selon l'invention utilisent des dérivés de formule (1) et/ou des sels hydrosolubles de ces dérivés.

De préférence, les sels hydrosolubles des dérivés de formule (1) sont choisis parmi les chlorures, les bromures, les fluorures et les iodures.

De préférence, les dérivés de formule (1) et leurs sels hydrosolubles sont choisis parmi les composés suivants :

- le chlorhydrate de dihydro-thiophen-2-ylidèneamine (chlorhydrate de 2-iminothiolane) :

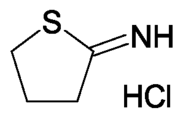

- le chlorhydrate de 5-méthyl-dihydro-thiophen-2-ylidèneamine :

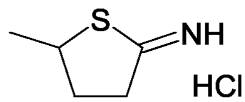

- le chlorhydrate de 5-phényl-dihydro-thiophen-2-ylidèneamine :

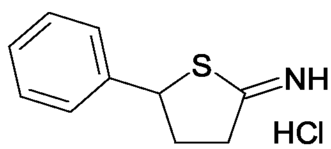

- le chlorhydrate de 5-ter-butyl-dihydro-thiophen-2-ylidèneamine :

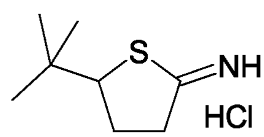

- le chlorhydrate de 5,5-diméthyl-dihydro-thiophen-2-ylidèneamine :

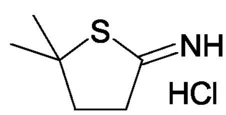

- le chlorhydrate de tétrahydro-2H-thiopyran-2-imine :

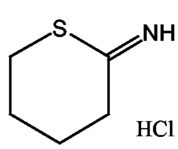

Avantageusement, le ou les dérivés de formule (1) et/ou le ou les sels hydrosolubles desdits dérivés représentent de 0,1 à 5%, de préférence 0,5 à 2%, en poids du poids total de la composition.
Le ou les dérivés de formule (1) et/ou leurs sels hydrosolubles doivent être placés dans un milieu cosmétiquement acceptable.
Pour cela, on choisit généralement un ou plusieurs solvants classiquement utilisés dans les compositions cosmétiques. On peut citer l'eau, les alcools en $C_1$-$C_6$, l'alcool benzylique, les éthers en $C_2$-$C_6$, et les esters en $C_2$-$C_6$.
De préférence, les alcools sont choisis parmi les alcanols, les alcanediols et l'alcool benzylique. Comme alcanols utilisables, on peut citer l'éthanol, le propanol ou l'isopropanol. Comme alcanediols utilisables, on peut citer

l'éthylène glycol, le propylène glycol et le pentanediol.

Par exemple, le solvant peut être un mélange eau/alcool.

La composition utilisée dans les procédés selon l'invention et comprenant le ou les dérivés de formule (1) et/ou leurs sels hydrosolubles doit avoir un pH neutre.

Par pH neutre, on entend un pH allant de 6 à 8, de préférence de 6,5 à 7,5 et mieux de 7 à 7,5.

Pour cela, le pH est généralement stabilisé à l'aide d'une solution tampon.

Par solution tampon, on entend une solution dont le pH ne varie pas par dilution ou par ajout d'une faible quantité de base ou d'acide.

La formulation des solutions tampons est bien connue de l'homme du métier.

Dans les procédés selon l'invention, on utilise avantageusement une solution tampon phosphate, par exemple une solution tampon à base de phosphate monopotassique $KH_2PO_4$.

La composition utilisée selon l'invention peut également comprendre au moins un adjuvant cosmétiquement acceptable classiquement utilisé dans les compositions cosmétiques destinées à être appliquées sur les cheveux, et qui ne nuit pas aux propriétés recherchées.

On peut citer par exemple les gélifiants et/ou épaississants, les tensioactifs anioniques, non ioniques, cationiques ou amphotères, les agents propénétrants, les émulsionnants, les parfums, les conservateurs, les charges, les filtres solaires, les matières colorantes, les protéines, les vitamines, les provitamines, les polymères non fixants anioniques, non ioniques, cationiques ou amphotères, les agents hydratants, les émollients, les agents adoucissants, les huiles minérales, végétales ou synthétiques, les actifs hydrophiles ou lipophiles comme les céramides et les pseudocéramides, les agents anti-mousse, les agents antiperspirants, les agents anti-radicaux libres, les polymères fixants ou non, les agents bactéricides, et les agents anti-pelliculaires.

La composition utilisée selon l'invention peut se présenter sous la forme d'une lotion, épaissie ou non, d'une crème, épaissie ou non, d'un gel ou de toute autre forme appropriée. Elle peut éventuellement être conditionnée dans un flacon pompe ou dans un récipient aérosol.

La composition utilisée dans les procédés selon l'invention est appliquée sur les cheveux pendant une durée suffisante pour obtenir l'effet recherché. Cette durée peut aller de 10 minutes à 3 heures. Généralement, la composition est appliquée pendant 1,5 heures à 2,5 heures.

De même, la composition utilisée dans les procédés selon l'invention est généralement appliquée sur les cheveux à une température comprise entre 20°C et 70°C, de préférence entre 40°C et 60°C.

La présente invention est illustrée par les exemples suivants.

Exemple 1

On cherche à mesurer l'efficacité de la mise en forme obtenue par un procédé de traitement selon l'invention.

1. On prépare une solution comprenant 1% en poids de chlorhydrate de 2-iminothiolane (commercialisé par la société ALDRICH CHEM CO sous la référence 33,056-6) dans un tampon phosphate.

La composition du tampon phosphate est la suivante :

- tampon phosphate à 0,05M (6,8g de $KH_2PO_4$ - PROLABO NORMA PUR)
- eau qsp 200 ml
- KOH à 2N qsp pH = 7,84.

Une mèche de cheveux naturels 90% blancs est entourée autour d'un bigoudi et introduite dans cette solution, la solution étant en une quantité telle que le rapport de bain soit de 10 mL de solution pour 1 gramme de cheveux. Le pH final de la solution est de 7,4.

On laisse poser à 50°C pendant 2 heures.

Après rinçage, une étape de fixation est réalisée sur les cheveux précédemment traités : une solution d'eau oxygénée 8 volumes à pH égal à 3 (obtenu par introduction d'acide citrique) est appliquée pendant 10 minutes à 30°C. Le rapport de bain est de 10 mL de solution oxydante pour 1 gramme de cheveux.

2. Un traitement comparatif de mise en forme permanente des cheveux selon l'état de la technique est réalisé sur des cheveux 90% blancs issus du même lot que précédemment.

Une mèche de cheveux est enroulée sur un bigoudi et subit une étape de réduction par une solution aqueuse contenant 1 mol/L d'acide thioglycolique et de l'hydroxyde d'ammonium en quantité suffisante pour obtenir un pH alcalin de 8,5. Le rapport de bain est de 10 mL de solution pour 1 gramme de cheveux.

On laisse poser à 30°C pendant 15 minutes.

Après rinçage, une étape d'oxydation est réalisée sur les cheveux : une solution d'eau oxygénée 8 volumes

à pH égal à 3 (obtenu par introduction d'acide citrique) est appliquée pendant 10 minutes à 30°C. Le rapport de bain est de 10 mL de solution oxydante pour 1 gramme de cheveux.

3. Une mèche de cheveux 90% blancs issus du même lot que précédemment est enroulée sur un bigoudi, mais ne subit aucun traitement. Cette mèche sert de témoin.

Les 3 mèches bouclées précédemment préparées sont rincées, séchées puis introduites dans une boîte à gants à humidité relative contrôlée (45%) et à température contrôlée (25°C), pendant 12 heures. Les bigoudis sont retirés des mèches qui sont ensuite disposées dans une boîte à gants à humidité relative contrôlée (80%) et à température contrôlée (25°C) pendant 1 heure. La longueur de la boucle (définie par le diamètre de la portion bouclée, en millimètres) mesurée à t=0, t=30 minutes et t=1 heure permet de quantifier la détente des cheveux et donc de caractériser la durabilité du traitement appliqué. Les résultats sont présentés dans le tableau 1.

Tableau 1

| | Cheveux témoins | Cheveux traités par l'acide thioglycolique pH 8,5 | Cheveux traités par le chlorhydrate de 2-iminothiolane pH 7,4 |
|---|---|---|---|
| Longueur de boucle initiale (mm) | 8,5 | 7 | 7,5 |
| Longueur de boucle à t=30min (mm) | 15,5 | 9,5 | 12,5 |
| Longueur de boucle à t=1h (mm) | 16,5 | 12,5 | 14 |

Ces résultats montrent que le traitement par le chlorhydrate de 2-iminothiolane à pH neutre conduit à une mise en forme durable.

Exemple 2

On cherche dans cet exemple à évaluer la compatibilité de la mise en forme effectuée par le procédé de traitement selon l'invention avec une coloration d'oxydation.

On effectue une coloration d'oxydation sur les 3 mèches de cheveux préparées selon l'exemple 1, c'est-à-dire la mèche traitée par le chlorhydrate de 2-iminothiolane, la mèche traitée par l'acide thioglycolique et la mèche témoin.

La coloration est effectuée en utilisant la formule commerciale de L'OREAL Professionnel Crescendo 7.44 selon le protocole décrit dans la notice d'utilisation du produit.

Le mélange est appliqué sur les cheveux dans un rapport de bain de 10g de composition pour 1g de cheveux. Les mèches sont traitées 30 minutes à 30°C puis rincées, lavées par une solution shampooing Dop® Camomille à 2% dans l'eau, et séchées à température ambiante.

La couleur des mèches est alors mesurée à l'aide d'un spectrocolorimètre Minolta CM2022 afin d'évaluer la montée de la couleur dans chaque cas. Les mesures sont réalisées dans l'espace couleur L*a*b*.

La montée de la couleur ΔE est quantifiée par la valeur de l'écart couleur ΔE*ab définie par l'équation suivante :

$$\Delta E^*ab = [\, (\Delta L^*)^2 + (\Delta a^*)^2 + (\Delta b^*)^2 \,]^{1/2}.$$

Les résultats sont présentés dans le tableau 2.

Tableau 2

| | Cheveux naturels | Cheveux traités par l'acide thioglycolique pH 8,5 | Cheveux traités par le chlorhydrate de 2-iminothiolane pH 7,4 |
|---|---|---|---|
| Montée de la couleur (ΔE) | 19,8 +/- 1,0 | 29,0 +/- 0,5 | 23,9 +/- 1,4 |

Ces résultats montrent que le traitement par le chlorhydrate de 2-iminothiolane à pH neutre permet de diminuer la montée de la couleur Crescendo 7.44 et de réduire ainsi l'écart avec le cheveu naturel n'ayant pas fait l'objet d'un traitement de mise en forme.

Cela traduit une meilleure compatibilité d'un traitement de mise en forme par le chlorhydrate de 2-iminothiolane avec une coloration d'oxydation par rapport au traitement à l'acide thioglycolique.

Exemple 3

On cherche dans cet exemple à évaluer le renforcement des cheveux apporté par le traitement au 2-iminothiolane.

1. On prépare une solution comprenant 1% en poids de chlorhydrate de 2-iminothiolane (commercialisé par la société ALDRICH CHEM CO sous la référence 33,056-6 ) dans un tampon phosphate.
La composition du tampon phosphate est la suivante :

- tampon phosphate à 0,05M (6.8g de $KH_2PO_4$ - PROLABO NORMA PUR)
- eau qsp 200 ml
- KOH à 2N qsp pH = 7,84.

Une mèche de cheveux naturels 90% blancs est introduite dans cette solution, la solution étant en une quantité telle que le rapport de bain soit de 10 mL de solution pour 1 gramme de cheveux. Le pH final de la solution est de 7,4.

On laisse poser à 50°C pendant 2 heures.

Après rinçage, une étape de fixation est réalisée sur les cheveux précédemment traités : une solution d'eau oxygénée 8 volumes à pH égal à 3 (obtenu par introduction d'acide citrique) est appliquée pendant 10 minutes à 30°C. Le rapport de bain est de 10 mL de solution oxydante pour 1 gramme de cheveux.

2. Une mèche de cheveux 90% blancs issus du même lot que précédemment ne subit aucun traitement. Cette mèche sert de témoin.

Les 2 mèches précédemment préparées sont rincées, séchées puis introduites dans une boîte à gants à humidité relative contrôlée (45%) et à température contrôlée (25°C), pendant 12 heures.

Un panel d'experts a jugé les cheveux traités au 2-iminothiolane renforcés par rapport aux cheveux non traités.

**Revendications**

1. Procédé pour renforcer les cheveux, **caractérisé en ce qu'**il comprend une étape de traitement des cheveux par une composition de pH neutre comprenant, dans un milieu cosmétiquement acceptable, au moins un dérivé du 2-iminothiolane de formule (1) :

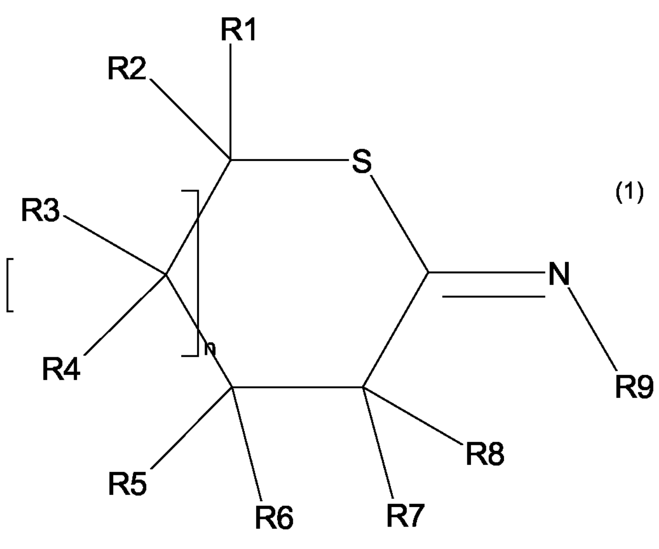

(1)

où

R1 à R8 représentent indépendamment soit un atome d'hydrogène, soit une chaîne hydrocarbonée linéaire ou ramifiée en $C_1$-$C_4$, présentant éventuellement une ou plusieurs insaturations, éventuellement substituée par un ou plusieurs groupes choisis parmi les groupes thiol, thioalkyle en $C_1$-$C_4$, alkylthiol en $C_1$-$C_4$, N,N-dialkylamine en $C_2$-$C_8$, alkylcarbonylthiol en $C_2$-$C_4$, ou N-alkylcarbonylamine en $C_2$-$C_4$, soit un groupe aryle en $C_6$ éventuellement substitué par un ou plusieurs groupes choisis parmi les groupes halogéno, thiol ou alkyle linéaire ou ramifié en $C_1$-$C_4$,
R9 désigne un atome d'hydrogène ou une chaîne hydrocarbonée linéaire ou ramifiée en $C_1$-$C_4$,
n est égal à 0 ou 1 ;
et/ou au moins un sel hydrosoluble desdits dérivés de formule (1).

2. Procédé selon la revendication 1 **caractérisé en ce qu'**il est mis en oeuvre indépendamment de tout autre traitement capillaire.

3. Procédé selon la revendication 1 **caractérisé en ce qu'**il est mis en oeuvre en association avec un autre traitement capillaire, de préférence avant, pendant ou après ledit traitement.

4. Procédé selon la revendication 3 **caractérisé en ce que** ledit traitement capillaire est choisi parmi le shampooing, l'après-shampooing, la mise en forme permanente ou semi-permanente, le défrisage et le coiffage des cheveux.

5. Procédé pour améliorer la compatibilité d'un traitement de mise en forme des cheveux avec un traitement cosmétique ultérieur, autre qu'un traitement de mise en forme des cheveux, comprenant une étape initiale de réduction et une étape ultérieure de fixation, **caractérisé en ce qu'**il consiste à remplacer l'étape initiale de réduction par une étape de traitement avec une composition de pH neutre comprenant, dans un milieu cosmétiquement acceptable, au moins un dérivé du 2-iminothiolane de formule (1) :

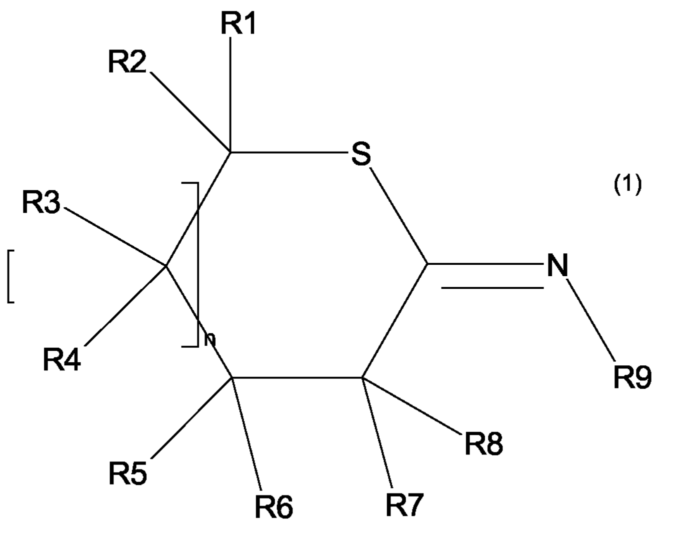

(1)

où
R1 à R8 représentent indépendamment soit un atome d'hydrogène, soit une chaîne hydrocarbonée linéaire ou ramifiée en $C_1$-$C_4$, présentant éventuellement une ou plusieurs insaturations, éventuellement substituée par un ou plusieurs groupes choisis parmi les groupes thiol, thioalkyle en $C_1$-$C_4$, alkylthiol en $C_1$-$C_4$, N,N-dialkylamine en $C_2$-$C_8$, alkylcarbonylthiol en $C_2$-$C_4$, ou N-alkylcarbonylamine en $C_2$-$C_4$, soit un groupe aryle en $C_6$ éventuellement substitué par un ou plusieurs groupes choisis parmi les groupes halogéno, thiol ou alkyle linéaire ou ramifié en $C_1$-$C_4$,
R9 désigne un atome d'hydrogène ou une chaîne hydrocarbonée linéaire ou ramifiée en $C_1$-$C_4$,
n est égal à 0 ou 1 ;
et/ou au moins un sel hydrosoluble desdits dérivés de formule (1).

6. Procédé selon la revendication 5 **caractérisé en ce que** l'étape ultérieure de fixation est effectuée soit par mise en contact des cheveux avec l'oxygène de l'air, soit par application d'une composition oxydante comprenant au moins un agent oxydant.

7. Procédé selon la revendication 6 **caractérisé en ce que** l'agent oxydant est choisi parmi l'eau oxygénée et les bromates alcalins, de préférence l'eau oxygénée.

8. Procédé selon l'une quelconque des revendications 5 à 7 **caractérisé en ce que** le traitement cosmétique ultérieur est une coloration d'oxydation.

9. Procédé selon la revendication 8 **caractérisé en ce que** la coloration d'oxydation est effectuée en utilisant un ou plusieurs colorants d'oxydation choisis parmi les bases d'oxydation, les orthodiphénols, les coupleurs et leurs mélanges.

10. Procédé selon l'une quelconque des revendications précédentes **caractérisé en ce que** les sels hydrosolubles des dérivés de formule (1) sont choisis parmi les chlorures, les bromures, les fluorures et les iodures.

11. Procédé selon l'une quelconque des revendications précédentes **caractérisé en ce que** les dérivés de formule (1) et leurs sels hydrosolubles sont choisis parmi les composés suivants :

- le chlorhydrate de dihydro-thiophen-2-ylidèneamine (chlorhydrate de 2-iminothiolane) :

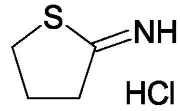

- le chlorhydrate de 5-méthyl-dihydro-thiophen-2-ylidèneamine :

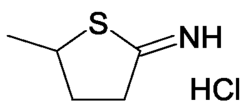

- le chlorhydrate de 5-phényl-dihydro-thiophen-2-ylidèneamine :

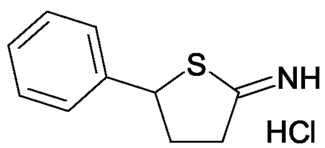

- le chlorhydrate de 5-ter-butyl-dihydro-thiophen-2-ylidèneamine :

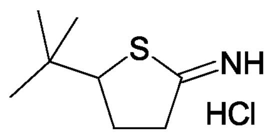

- le chlorhydrate de 5,5-diméthyl-dihydro-thiophen-2-ylidèneamine :

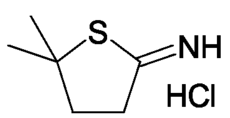

- le chlorhydrate de tétrahydro-2H-thiopyran-2-imine :

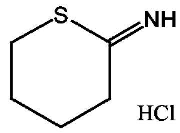

**12.** Procédé selon l'une quelconque des revendications précédentes **caractérisé en ce que** le ou les dérivés de formule (1) et/ou le ou les sels hydrosolubles desdits dérivés représentent de 0,1% à 5%, de préférence de 0,5% à 2%, en poids du poids total de la composition.

**13.** Procédé selon l'une quelconque des revendications précédentes **caractérisé en ce que** la composition comprend au moins un solvant choisi parmi l'eau, les alcools en $C_1$-$C_6$, l'alcool benzylique, les éthers en $C_2$-$C_6$, les esters en $C_2$-$C_6$.

**14.** Procédé selon la revendication 13 **caractérisé en ce que** les alcools sont choisis parmi les alcanols, les alcanediols et l'alcool benzylique.

**15.** Procédé selon la revendication 14 **caractérisé en ce que** les alcanols sont choisis parmi l'éthanol, le propanol ou l'isopropanol.

**16.** Procédé selon la revendication 14 **caractérisé en ce que** les alcanediols sont choisis parmi l'éthylène glycol, le propylène glycol et le pentanediol.

**17.** Procédé selon l'une quelconque des revendications 12 à 14 **caractérisé en ce que** le solvant est un mélange eau/alcool.

**18.** Procédé selon l'une quelconque des revendications précédentes **caractérisé en ce que** le pH de la composition est compris entre 6,5 et 7,5.

**19.** Procédé selon la revendication 18 **caractérisé en ce que** le pH de la composition est stabilisé par une solution tampon, de préférence une solution tampon phosphate.

**20.** Procédé selon l'une quelconque des revendications précédentes **caractérisé en ce qu'**elle comprend un ou plusieurs adjuvants cosmétiques choisis parmi les gélifiants et/ou épaississants, les tensioactifs anioniques, non ioniques, cationiques ou amphotères, les agents propénétrants, les émulsionnants, les parfums, les conservateurs, les charges, les filtres solaires, les matières colorantes, les protéines, les vitamines, les provitamines, les polymères non fixants anioniques, non ioniques, cationiques ou amphotères, les agents hydratants, les émollients, les agents adoucissants, les huiles minérales, végétales ou synthétiques, les actifs hydrophiles ou lipophiles comme les céramides et les pseudocéramides, les agents anti-mousse, les agents antiperspirants, les agents anti-radicaux libres, les polymères fixants ou non, les agents bactéricides, et les agents anti-pelliculaires.

**21.** Procédé selon l'une quelconque des revendications précédentes **caractérisé en ce que** la composition se présente sous la forme d'une lotion, d'une crème ou d'un gel.

**22.** Procédé selon l'une quelconque des revendications précédentes **caractérisé en ce que** la composition est appliquée sur les cheveux pendant une durée comprise entre 10 minutes et 3 heures, de préférence entre 1,5 heures et 2,5 heures.

**23.** Procédé selon l'une quelconque des revendications précédentes **caractérisé en ce que** la composition est appliquée sur les cheveux à une température comprise entre 20°C et 70°C, de préférence entre 40°C et 60°C.

Office européen des brevets

# RAPPORT DE RECHERCHE EUROPEENNE

Numéro de la demande
EP 05 30 0070

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.7) |
|---|---|---|---|
| D,X | KUZUHARA A: " CHEMICAL MODIFICATION OF KERATIN FIBERS USING 2-IMINOTHIORANE HYDROCHLORIDE " JOURNAL OF APPLIED POLYMER SCIENCE, vol. 90, no. 13, 2003, pages 3646-3651, XP002295621 * le document en entier * ----- | 1-4, 10-23 | A61K7/06 |
| D,X | US 4 263 277 A (TRIPATHI UMA P ET AL) 21 avril 1981 (1981-04-21) * revendications 5,6 * ----- | 5-23 | |
| D,A | KUZUHARA A ET AL: "REDUCING WRINKLE FORMATION IN WOOL WITH 2-IMINOTHIORANE HYDROCHLORIDE" TEXTILE RESEARCH JOURNAL, TEXTILE RESEARCH INSTITUTE. PRINCETON, N.J, US, vol. 72, no. 4, avril 2002 (2002-04), pages 285-289, XP001111611 ISSN: 0040-5175 * abrégé * ----- | 1-23 | |

DOMAINES TECHNIQUES RECHERCHES (Int.Cl.7)

A61K

Le présent rapport a été établi pour toutes les revendications

| Lieu de la recherche | Date d'achèvement de la recherche | Examinateur |
|---|---|---|
| La Haye | 7 juin 2005 | Voyiazoglou, D |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
.......................................................................................................
& : membre de la même famille, document correspondant

2

EPO FORM 1503 03.82 (P04C02)

**ANNEXE AU RAPPORT DE RECHERCHE EUROPEENNE RELATIF A LA DEMANDE DE BREVET EUROPEEN NO.** EP 05 30 0070

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

07-06-2005

| Document brevet cité au rapport de recherche | Date de publication | Membre(s) de la famille de brevet(s) | Date de publication |
|---|---|---|---|
| US 4263277 A | 21-04-1981 | AUCUN | |

EPO FORM P0460

Pour tout renseignement concernant cette annexe : voir Journal Officiel de l'Office européen des brevets, No.12/82